Europäisches Patentamt

European Patent Office

Office européen des brevets

(11) Publication number: **0 298 768 B1**

(12) EUROPEAN PATENT SPECIFICATION

(45) Date of publication of patent specification: **11.05.94**

(21) Application number: **88306268.9**

(22) Date of filing: **08.07.88**

(51) Int. Cl.⁵: **A61K 9/28**, A61K 9/36, A61K 47/00, A61K 31/44, A61K 31/135

(54) **Flavoured film-coated tablet.**

(30) Priority: **09.07.87 US 71635**

(43) Date of publication of application:
**11.01.89 Bulletin 89/02**

(45) Publication of the grant of the patent:
**11.05.94 Bulletin 94/19**

(84) Designated Contracting States:
**AT BE CH DE ES FR GB GR IT LI LU NL SE**

(56) References cited:
EP-A- 0 058 765      CH-A- 509 804
DE-B- 2 045 749      FR-A- 1 273 374
GB-A- 1 396 113      US-A- 3 146 169
US-A- 3 256 111      US-A- 4 302 440

(73) Proprietor: **THE WELLCOME FOUNDATION LIMITED**
**Unicorn House**
**160 Euston Road**
**London NW1 2BP(GB)**

(72) Inventor: **McCabe, Terrance Thomas**
**112 Weathersfield Drive**
**Durham North Carolina 27713(US)**
Inventor: **Stagner, Robert Allen**
**300 Ravenwood Drive**
**Greenville North Carolina 27834(US)**
Inventor: **Sutton, Joel Elmore, Jr.**
**1, Dogwood Court**
**Greenville North Carolina 27858(US)**

(74) Representative: **Rollins, Anthony John et al**
**Group Patents & Agreements**
**The Wellcome Research Laboratories**
**Langley Court**
**Beckenham Kent BR3 3BS (GB)**

**Description**

The present invention relates to a thinly-coated pharmaceutical tablet and to methods of its preparation. In particular, the invention relates to a flavoured, sweetened film-coated tablet, and especially to such tablets containing unpleasant tasting ingredients, such as triprolidine hydrochloride or pseudoephedrine hydrochloride.

Thin film coating of pharmaceutical tablets allows efficient, controlled, uniform and reproducible coats. Use of multiple layers of coating, such as the polymeric undercoat, polymeric pigmented second coat and polymeric finish coat allows the preparation of very smooth glossy tablets (Ohno, U.S. Pat. No. 4,001,390).

Numerous methods for pan-coating pharmaceutical tablets have been developed and are summarised in Pharmaceutical Dosage Forms: Tablets, Volume 3 (eds. Lieberman and Lachman, 1982, Marcel Dekker). They include sugar-coating techniques, solvent film coating, aqueous film coating, delayed release coating, and granule coating. Pulverized medicine may also be wrapped in a transparent, glossy, resistant, soluble or semi-permeable film as provided by Motoyama et al. (U.S. Pat. No. 4,154,636).

Pharmaceutical tablets have been coated for a variety of reasons, including masking objectionable flavours or odours, protecting unstable tablet compositions, providing protection of the tablet through the stomach with enteric coatings, improving the appearance of the tablet or separating medicine ingredients into a core segment and coating segment.

Aspirin tablets or other tablets that are powdery, easily dissolved and friable have been treated with a variety of coatings to keep them from dissolving too soon (John et al., U.S. Pat. No. 4,302,440). Also, other polymers in non-aqueous vehicles have been used to granulate tablets (Gans et al., U.S. Pat. No. 3,388,041) or to coat onto tablets (Jeffries, U.S. Pat. No. 3,149,040 and Eisai, U.K. 1396113) to protect from dissolving in the stomach or to delay the drug's release. Other non-aqueous film-coating systems have been designed to be applied to a variety of tablets containing a variety of active ingredients as illustrated by Singiser, U.S. Pat. No. 3,256,111 and Brindamour, U.S. Pat. No. 3,383,236. The aqueous coating processes are environmentally more safe than the non-aqueous processes, which involve the use of organic solvents in film-coating solutions. Thin film coatings, which do not alter the dissolution characteristics of the tablet, may be readily formed using aqueous film-coating processes. Unless adequately thick or insoluble coatings are used, most coatings are not capable of effectively masking the strong objectionable bitter taste of triprolidine hydrochloride or other compounds with similar properties.

Previous attempts to solve the problem of masking the taste and odour of active ingredients in tablet form have led to slow-dissolving coatings, thicker coatings, and sugar coatings (sucrose or mannitol). Although an unflavoured soluble film-coating may normally be adequately thick to mask effectively the objectionable bitter taste of triprolidine and other compounds with similar properties, persons who have difficulty swallowing such tablets may find that even tablets having adequately thick soluble film-coatings may partially dissolve in the mouth, thus decreasing the effectiveness of the coating in masking the objectionable flavour.

Tablets have been coated with compositions containing sugar or sugar substitutes to make them more palatable as well as to improve their appearance in some cases. One sugar-coating pan process involves applying a first water-repellent layer, a subcoat and a sugar coat, and colouring and polishing the sugar-coated tablet. The sugar-coating pan process is time-consuming and greatly increases the tablet size. It is believed that the prior sugar coatings do not include use of strong pleasant masking flavours to better disguise the bitter taste.

Although it is believed that strong, masking flavourings such as fruit or mint flavourings have not been used with tablet coatings, some flavourings have been used in liquid medicines. Liquid medicines having strong tastes have been mixed with sweet and/or flavoured substances such as fruit flavours to mask the taste. For other oral, solid dosage forms, medicinal compounds have been mixed with waxy materials and water-swellable high molecular weight materials to mask objectionable tastes.

It is believed that the previous uses of flavourings or fragrances in thin-film coatings for pharmaceutical tablets have not utilized aqueous spray coatings and have included mild flavoured or low concentrations of flavoured ingredients having pronounced, characteristic fragrances for relatively mild-flavoured medicines, especially those having an objectionable odour. Such flavoured or fragrant coatings include a pressed film coating incorporating 0.5% orange essence to impart the smell of an orange (Motoyama, U.S. Pat. No. 4,154,636) and a non-aqueous air spray coating containing a 5.2% ethyl vanillin on a vitamin tablet core (Singiser, U.S. Pat. No. 3,256,111). An aqueous film coating for aspirin in which unspecified flavourings were mentioned as optional additions is found in John, U.S. Pat. No. 4,302,440.

Another major function of tablet coatings has been to aid in tablet identification. Thus, the use of coatings containing pigments on tablets provides a way to identify tablets by colour. Pigment addition also allows the tablets to have a more uniform and pleasing appearance. Tablet coatings comprising a coloured film coating have been prepared, for example, by dispersing an anhydrous pigment suspension in a polymer solution (Signorino U.S. Pat. No. 3,981,984). However, persons with impaired vision often have difficulty in being sure that they are taking the correct medicine even with colour-coded tablets.

The present invention provides an unexpected advantage of masking unpleasant medicinal tastes such as that associated with triprolidine through the use of distinctive flavouring agents in combination with a sweetening agent in the aqueous coating dispersion. Thus, one object of this invention is to provide a pharmaceutical tablet comprising an unpleasant tasting, solid core and a flavoured, pharmaceutically acceptable, thin film coating, said coated tablet showing a weight increase of 0.5 to 15 parts per 100 parts by weight of the core, said coating comprising a water-soluble, film-forming polymer, a volatile flavouring agent and a sweetening agent, said coating being capable of masking the unpleasant taste of the core, and said coating having a flavour that is retained in the coating for at least 24 months during storage and that provides a taste perception of said flavour for at least five seconds after oral administration of said tablet. Not only does the film coating of the invention hide an objectionable taste, but it also provides a perceptible pleasing taste to the tablet. This acceptable or pleasant taste component in the coating , in addition to the masking effect provided by the presence of the coating itself, is more effective than a coating by itself, in removing and covering unpleasant tastes. The flavoured coating of the invention also provides a pleasant taste advantage even if the core tablet itself is neutral-tasting and does not have an objectionable taste.

Another object of the invention is to provide a coated pharmaceutical tablet that will enable oral identification of the tablet due to the particular flavour of the coat being associated with the particular core tablet composition. Oral flavour identification of this invention allows visually impaired as well as other persons to know that the correct medication is being taken so that mistakes in medication may be avoided.

Another object of the invention is to provide a coated pharmaceutical tablet that enables different strengths of the same active ingredient, such as a prescription medicine, to be identified by different flavoured coatings being applied to the different ingredient strengths.

Another object of the invention is to provide a coated pharmaceutical tablet that enables increased compliance with prescribed medicine schedules. The flavoured coat provides a flavoured oral stimulus that enables those who have taken flavoured-coated tablets to have an enhanced memory of having taken the tablet through remembrance of the particular flavour of coating. The flavoured coating of the invention also enhances the appeal of a particular medicine so that persons do not avoid taking their medicine.

Another object of the invention is to provide a smooth easily swallowed tablet and to facilitate swallowing ease through increased salivation if the coated tablet lingers in the mouth and is tasted.

Another object of the invention is to provide a coated pharmaceutical tablet that does not slow the dissolution of the core tablet and in which the bioavailability of the active ingredients is not significantly reduced or impaired.

Another object of the invention is to provide a process for preparing a flavour-coated pharmaceutical tablet comprising an aqueous coating process, which is less hazardous to the environment than a non-aqueous coating process.

Another object of the invention is to provide a coated pharmaceutical tablet to reduce the potential for dust generation inherent in uncoated tablets.

Another object of the invention is to provide a flavour-coated pharmaceutical tablet in which the flavour is retained for the anticipated shelf life of the core tablet.

Still other objects and advantages of the invention will be apparent to those of skill in the art after reading the following description.

Thus the present invention relates to a flavoured thin film coating on solid oral dosage pharmaceuticals, in particular, those containing unpleasant-tasting active ingredients such as triprolidine hydrochloride. The method of the invention comprises applying a water-soluble, pharmaceutically-acceptable polymeric coating such as a hydroxypropyl methylcellulose coating containing a flavouring agent and a sweetening agent onto the exterior surfaces of the tablet.

In one aspect, therefore, the invention provides a pharmaceutical tablet comprising an unpleasant tasting, solid core and a flavoured, pharmaceutically acceptable, thin film coating, said coated tablet showing a weight increase of 0.5 to 15 parts per 100 parts by weight of the core, said coating comprising a water-soluble, film-forming polymer, a volatile flavouring agent and a sweetening agent, said coating being capable of masking the unpleasant taste of the core, and said coating having a flavour that is retained in the coating for at least 24 months during storage and that provides a taste perception of said flavour for at least five seconds alter oral administration of said tablet.

In another aspect, the invention provides a method for preparing a flavoured, film-coated pharmaceutical tablet as described in the preceding paragraph which comprises spray-coating pharmaceutical core tablets with a thin film coating characterised in that the coating mixture contains a flavouring agent and a sweetener. The coating is preferably applied continuously (i.e. not intermittently).

The method of the invention may be effected using standard pharmaceutical aqueous spray coating techniques and conditions, using a flavoured coating.

In a preferred embodiment the method of the invention is carried out as follows.

An aqueous dispersion comprising a film-coating substance, a flavouring agent and a sweetening agent is prepared. Suitably formulated core tablets are placed in a coating chamber. A preferred composition of coating material, of an excessive volume to allow coating losses to the pan, exhaust and spray equipment, is sprayed into the coating chamber until the coated tablets show a weight increase of 0.5 to 15.0 parts per 100 parts by weight of the core tablet weight. It will be appreciated that the spray-coating of the aqueous dispersion onto the exterior surface of the core tablets will be effected at a pan rotation speed and under airflow and temperature conditions sufficient to enable evaporation of the water and even-coating of the core tablets. The skilled practitioner in pharmaceutical art will readily be able to select the optimum conditions on the basis of routine experimentation. The preferred method of the invention comprises a one-step continuous spray-coating process to apply the thin flavoured coating. Thus, the preferred embodiment is distinguishable from sugar-coating processes in which multiple layers of sugar-containing coating are applied, each followed by a drying period. It is also possible to apply more than one flavoured coat or to apply the flavoured coating after an initial sealing coat. If any coating, such as a wax coating, is applied after the flavoured coating, it must be designed to allow taste perception of the flavoured coating.

Application of the film-coat by spray-coating produces a tablet with a seamless film-coat. This is in contrast to tablets which are coated by wrapping them in pre-formed films (eg. as described in Motoyama, US patent no. 4, 154,636). Such tablets will contain a seam which may be a potential point of weakness, at which the film coat may break, thereby reducing or negating benefits of the film-coat. A further advantageous feature of the present invention therefore is that the tablets have a seamless, flavoured film-coat.

The preferred pharmaceutical tablet with which the flavoured coating of this invention is used contains triprolidine hydrochloride and pseudoephedrine hydrochloride. These tablets contain from 12 to 300mg pseudoephedrine hydrochloride per tablet and 0.5 to 12.5mg triprolidine hydrochloride per tablet with the amounts of the active ingredients present in the tablets of the cited examples being 60 and 2.5mg, respectively, in a typical 150mg tablet. The coating increases the weight of the tablets by an average of 5%. Tablets containing either pseudoephedrine hydrochloride or triprolidine hydrochloride as the only active ingredient, also may be flavour-coated. The advantages of this invention are also realized through flavour-coating of other bitter or objectionably strong flavoured tablets, especially those that bleed through the thin coating. Such other bitter or objectionable-tasting active ingredients include, but are not limited to, trimethoprim, sulfamethoxazole, guaifenesin, chlorpheniramine maleate, dextromethorphan, bupropion, azidothymidine and other salts or combinations of these ingredients and those of the preferred embodiment. The invention may also be used with sustained-release formulations.

The preferred film coating of this invention is comprised of a commercial film-coating product designed for aqueous film coating containing the water-soluble, film-forming resin, hydroxypropyl methylcellulose and polyethylene glycol (or other suitable plasticizing agents such as propylene glycol or glycerine) and optionally containing titanium dioxide (or other colourant or opacifying agent). Such a product is commercially available under the trade name Opadry White (TM) (Colorcon, West Point, Pennsylvania). A suitable blend comprises 0 to about 20% w/w titanium dioxide or colourant, about 5 to about 95% w/w hydroxypropyl methylcellulose, and 0 to about 25% w/w polyethylene glycol. The most preferred embodiment comprises 10.5% non-water additives, of which 7.5% is Opadry. Therefore, most of the weight of the non-water additives of the coating dispersion is comprised of Opadry. More than 25% Opadry makes the coating too thick to spray easily while concentrations that are too low decrease the efficiency of coating. This blend plus flavouring and sweetening agents is added to purified water at ambient temperature in a vortex mixer such as a Lightnin Mixer Model V-7 (Mixing Equipment Co., Rochester, New York). Other Opadry coating products such as Opadry Clear or Opadry with various pigment lakes may also be used in the invention to change the appearance of the tablets without adversely affecting the flavour characteristics of the invention. Other aqueous film-forming polymers may also be employed in place of hydroxypropyl methylcellulose.

Small amounts of a flavouring agent and a sweetening agent are added so that the total percent of the components added to the water is 2 to 25% w/w based on the weight of the total dispersion. Flavourings may be obtained from a variety of sources with the relevant criteria being strength and pleasing nature of the flavour. Suitable flavourings for use in the present invention include fruit and mint flavourings. The flavour agent selected, the film coating dispersion formulation and the amount of solids sprayed on to the tablet affect the flavour strength of the desired product. The preferred flavouring amount is readily

EP 0 298 768 B1

determined by balancing the goal of adding an amount sufficient to mask the core tablet taste and provide a distinct, characteristic and pleasing taste, and the goal of keeping the tablet from being too much like a candy or mint product. The desired strength of the flavouring may vary depending on the type of tablet and the intended recipients and the identity of the flavouring.

The sweetening agent in the preferred embodiment is confectioners sugar, but other sweetening agents such as saccharin, aspartame, mannitol, sorbitol or others used in foods, may also be employed. The preferred amount of sweetening agent will be a function of the sweetening capacity of the sweetening agent. For example, since aspartame is reported to be 160 times as sweet as sucrose, proportionally less aspartame than sucrose would be used to achieve the flavoured, film-coated tablet of this invention. The preferred range of confectioners sugar is about 0.5 to about 10% based on the weight of the film coating. The more preferred range is 2.5 to 10%. Most preferred is 2.5%. Concentrations from about 2.5 to 10% sugar allow a thin coating of about 100 $\mu$ thickness to be applied by the method of the invention to achieve the desired results of the invention.

The following equipment was used in practicing the method of this invention as demonstrated in the examples. The coating pan was an ACCELA-COTA$^R$ (Thomas Engineering, Inc., Hoffman Estates, Illinois) having a 24-inch (60.96cm) perforated coating pan rotating at about 8 rpm and providing about 36.79 cu m/min (1300 cu ft/min) of inlet air at a temperature of 90°C. Tablet bed temperature was maintained at 45°C. Although 45°C is the optimum temperature, acceptable quality coatings may be obtained at tablet temperatures from 38-55°C. The spraying unit was an air-atomized Binks Model 460 spray gun with two guns per pan (Binks Manufacturing Co. Franklin Park, Illinois), operating at 344.5 KPa (50psi) hydraulic pressure. A Masterflex peristaltic pump (Cole-Parmer Instrument Co., Chicago, Illinois) with Model 7015 pumpheads and tubing was used to pump the dispersion formulation of the invention. Equipment to be used for scale-up operations would be obvious to a person skilled in the art of pharmaceutical coatings. For example, larger ACCELA-COTA pans of 48 or 60 inches would accommodate increased number of core tablets. It is also clear that the inlet air volume, rotation speed of the pan and temperature are interactive factors in coating operations and the cited parameters and equipment are for illustration purposes only and do not limit the invention. Although use of air spraying units results in more even coating of core tablets due to better droplet-size control, airless spraying units may also be utilized.

When the flavour-coated tablets as prepared by the method of this invention are administered to a recipient, the positive taste perception of the flavoured coat of the invention lasts on the tongue for at least five seconds, which is generally more than enough time for the tablet to be swallowed before the tablet's bitterness becomes objectionable. Because the flavours used in this invention are volatile, it would be expected that the high temperatures employed during manufacturing would cause the flavouring agents to volatilize during the spray-coating process and the flavours to be lost. The surprising and unexpected result in the actual practice of this invention is that when the flavouring agents are incorporated into the coating dispersion with a sweetener, the flavours are retained. In fact, the flavours continue to be retained and to remain strong for an unexpectedly long period. Core tablets containing triprolidine hydrochloride and pseudoephedrine hydrochloride coated by the method of the invention as exemplified in the examples below have been stored in blister packs at 30°C for 24 months. Taste tests on these stored flavour-coated tablets revealed that the coating flavour is retained for at least 24 months, the anticipated shelf life for the coated tablets.

The following examples illustrate the invention without limiting it to the examples. In particular, numerous strongly flavoured agents, such as other fruit flavours, other mint-related flavours and other natural and artificial flavours, may be employed in lieu of those in the examples.

EXAMPLE 1

A coating dispersion formulation of the following percentages (w/w) is prepared: Opadry White, 7.5; natural and artificial peppermint flavour (International Flavors and Fragrances, Inc., New York, NY), 0.5; confectioners sugar, NF, 2.5; and purified water, 89.5. Five (5) kg of core tablets, each containing the active ingredients, triprolidine hydrochloride (2.5 mg) and pseudoephedrine hydrochloride (60 mg) and a suitable binder, are placed in a 24-inch ACCELA-COTA rotating at 8 rpm. A coating dispersion is applied using an air-atomized sprayer and standard coating procedures. Tablets with this coating possess a pleasant peppermint flavour when tasted.

The dissolution results of individual tablets using the USP/Paddle method (50 rpm in 900 ml distilled water at 37°C) are shown in Table 1. The table shows the mean percent of the core tablet active ingredients dissolved over time for coated and uncoated tablets (lower half of table as compared to upper half of table) as well as the standard deviation (SD) and the relative standard deviation (RSD). The coating

5

did not impair dissolution of the tablet.

## TABLE 1

### PERCENT COMPOUND DISSOLVED

#### (Uncoated Tablets)

| Tablet | Pseudoephedrine HCl | | | Triprolidine HCl | | |
|---|---|---|---|---|---|---|
| | 15 min | 30 min | 45 min | 15 min | 30 min | 45 min |
| 1 | 97.8 | 98.2 | 98.0 | 94.1 | 96.2 | 96.5 |
| 2 | 96.7 | 96.5 | 96.4 | 90.7 | 91.6 | 93.1 |
| 3 | 99.1 | 99.0 | 99.5 | 92.3 | 93.0 | 95.2 |
| 4 | 94.4 | 97.6 | 98.8 | 88.8 | 92.9 | 93.5 |
| 5 | 100.5 | 100.2 | 101.3 | 90.1 | 92.6 | 91.6 |
| 6 | 91.7 | 95.9 | 98.2 | 83.5 | 92.2 | 94.5 |
| Mean | 96.7 | 97.9 | 98.7 | 89.9 | 93.1 | 94.1 |
| SD | 3.2 | 1.6 | 1.6 | 3.6 | 1.6 | 1.7 |
| % RSD | 3.3 | 1.6 | 1.7 | 4.0 | 1.7 | 1.8 |

### PERCENT COMPOUND DISSOLVED

#### (Coated Tablets)

| Tablet | Pseudoephedrine HCl | | | Triprolidine HCl | | |
|---|---|---|---|---|---|---|
| | 15 min | 30 min | 45 min | 15 min | 30 min | 45 min |
| 1 | 98.1 | 99.5 | 97.9 | 92.2 | 92.8 | 91.0 |
| 2 | 97.1 | 99.3 | 99.2 | 91.4 | 92.3 | 93.1 |
| 3 | 94.9 | 95.2 | 96.3 | 90.0 | 88.9 | 91.7 |
| 4 | 95.6 | 97.2 | 97.6 | 91.6 | 95.1 | 93.6 |
| 5 | 93.2 | 92.6 | 94.3 | 88.5 | 94.6 | 89.4 |
| 6 | 99.6 | 99.3 | 100.1 | 94.2 | 97.6 | 93.2 |
| Mean | 96.4 | 97.2 | 97.6 | 91.3 | 93.6 | 92.0 |
| SD | 2.3 | 2.8 | 2.1 | 2.0 | 3.0 | 1.6 |
| % RSD | 2.4 | 2.9 | 2.1 | 2.1 | 3.2 | 1.8 |

### EXAMPLE 2

A coating dispersion formulation of the following percentages (w/w) is prepared: Opadry White, 7.5; natural and artificial peppermint flavour, 2.0; confectioners sugar, NF, 10.0; and purified water, 80.5. Five (5) kg of core tablets, each containing the active ingredients, triprolidine hydrochloride (2.5 mg) and pseudoephedrine hydrochloride (60 mg) and a suitable binder, are placed in a 24-inch ACCELA-COTA

6

rotating at 8 rpm. A coating dispersion is applied using an air-atomized sprayer and standard coating procedures. Tablets with this coating possess a pleasant peppermint flavour when tasted.

Dissolution results of individual tablets using the USP/Paddle method at 50 rpm in 900 ml distilled water tablets at 37°C are shown in Table 2.

## TABLE 2

### PERCENT COMPOUND DISSOLVED

#### (Uncoated Tablets)

| Tablet | Pseudoephedrine HCl | | | Triprolidine HCl | | |
|---|---|---|---|---|---|---|
| | 15 min | 30 min | 45 min | 15 min | 30 min | 45 min |
| 1 | 103.7 | 104.0 | 103.7 | 102.8 | 103.8 | 106.8 |
| 2 | 96.0 | 97.0 | 97.5 | 99.3 | 97.2 | 100.2 |
| 3 | 98.9 | 99.7 | 98.6 | 99.0 | 101.0 | 99.7 |
| 4 | 99.5 | 99.4 | 99.7 | 99.8 | 100.7 | 100.0 |
| 5 | 100.8 | 101.4 | 101.1 | 97.0 | 98.4 | 98.1 |
| 6 | 105.9 | 105.4 | 106.1 | 101.0 | 96.6 | 101.5 |
| Mean | 100.8 | 101.2 | 101.1 | 99.9 | 99.6 | 101.1 |
| SD | 3.5 | 3.1 | 3.3 | 1.9 | 2.7 | 3.0 |
| % RSD | 3.5 | 3.1 | 3.2 | 1.9 | 2.7 | 3.0 |

### PERCENT COMPOUND DISSOLVED

#### (Coated Tablets)

| Tablet | Pseudoephedrine HCl | | | Triprolidine HCl | | |
|---|---|---|---|---|---|---|
| | 15 min | 30 min | 45 min | 15 min | 30 min | 45 min |
| 1 | 99.7 | 103.2 | 101.5 | 96.3 | 99.6 | 106.3 |
| 2 | 104.1 | 104.6 | 103.8 | 97.8 | 96.8 | 101.1 |
| 3 | 97.3 | 97.5 | 95.6 | 95.8 | 92.1 | 98.9 |
| 4 | 104.4 | 103.9 | 103.0 | 98.8 | 96.9 | 98.6 |
| 5 | 99.3 | 100.5 | 99.0 | 92.4 | 95.8 | 97.1 |
| 6 | 103.5 | 103.0 | 103.1 | 97.9 | 97.1 | 96.2 |
| Mean | 101.4 | 102.1 | 101.0 | 97.3 | 97.2 | 99.7 |
| SD | 3.0 | 2.7 | 3.2 | 1.1 | 1.3 | 3.6 |
| % RSD | 2.9 | 2.6 | 3.1 | 1.1 | 1.3 | 3.7 |

### EXAMPLE 3

A coating dispersion formulation of the following percentages (w/w) is prepared: Opadry White, 7.5; natural and artificial cherry marasque flavour, 2.0; confectioners sugar, NF, 10.0; and purified water, 80.5. Five (5) kg of core tablets, each containing the active ingredients, triprolidine hydrochloride (2.5 mg) and pseudoephedrine hydrochloride (60 mg) and a suitable binder, are placed in a 24-inch ACCELA-COTA rotating at 8 rpm. A coating dispersion is applied using an air-atomized sprayer and standard coating procedures. Tablets with this coating possess a pleasant cherry flavour when tasted.

EXAMPLE 4

A coating dispersion formulation of the following percentages (w/w) is prepared: Opadry Clear, 7.5; natural and artificial peppermint flavour, 2.0; confectioners sugar, NF, 10.0; and purified water, 80.5. Five (5) kg of core tablets, each containing the active ingredients, triprolidine hydrochloride (2.5 mg) and pseudoephedrine hydrochloride (60 mg) and a suitable binder, are placed in a 24-inch ACCELA-COTA rotating at 8 rpm. A coating dispersion is applied using an air-atomized sprayer using coating procedures that are standard. Tablets with this coating possess a pleasant peppermint flavour when tasted.

**Claims**
**Claims for the following Contracting States : AT, BE, CH, DE, FR, GB, IT, LI, LU, NL, SE**

1. A pharmaceutical tablet comprising an unpleasant tasting, solid core and a flavoured, pharmaceutically acceptable, thin film coating, said coated tablet showing a weight increase of 0.5 to 15 parts per 100 parts by weight of the core, said coating comprising a water-soluble, film-forming polymer, a volatile flavouring agent and a sweetening agent, said coating being capable of masking the unpleasant taste of the core, and said coating having a flavour that is retained in the coating for at least 24 months during storage and that provides a taste perception of said flavour for at least five seconds after oral administration of said tablet.

2. A pharmaceutical tablet as claimed in claim 1 characterised in that the flavoured coating is applied by spray coating an aqueous dispersion comprising a water soluble pharmaceutically acceptable film-forming substance, a volatile flavouring agent and a sweetening agent onto the exterior surface of the core tablets.

3. A pharmaceutical tablet as claimed in claim 1 or claim 2, wherein the film-forming substance comprises hydroxypropyl methylcellulose.

4. A pharmaceutical tablet as claimed in any of claims 1 to 3, wherein the flavouring agent comprises peppermint flavouring.

5. A pharmaceutical tablet as claimed in any of claims 1 to 3, wherein the flavouring agent comprises fruit flavouring.

6. A pharmaceutical tablet as claimed in any of claims 1 to 5, wherein the sweetening agent comprises confectioners sugar.

7. A pharmaceutical tablet as claimed in any of claims 1 to 6, wherein the film-forming substance further contains titanium dioxide.

8. A pharmaceutical tablet as claimed in any of claims 1 to 7, wherein the tablet core comprises triprolidine hydrochloride.

9. A pharmaceutical tablet as claimed in any of claims 1 to 7, wherein the tablet core comprises pseudoephedrine hydrochloride.

10. A pharmaceutical tablet as claimed in any of claims 1 to 7, wherein the tablet core comprises triprolidine hydrochloride and pseudoephedrine hydrochloride.

11. A pharmaceutical tablet as claimed in any of claims 2 to 10, characterised in that:
    (a) the film-coating substance comprises hydroxypropyl methylcellulose, titanium dioxide and polyethylene glycol; and
    (b) the aqueous dispersion is comprised of about 7.5 % film-forming substance, about 0.5% flavouring agent, and about 2.5% sweetening agent.

**12.** A method for preparing a flavoured film-coated pharmaceutical tablet as claimed in any of claims 2 to 10 which comprises spray-coating pharmaceutical core tablets with a thin film coating, characterised in that the coating mixture comprises a water soluble pharmaceutically acceptable film forming agent, a volatile flavouring agent and a sweetening agent.

**Claims for the following Contracting States : ES, GR**

**1.** A method for preparing a pharmaceutical tablet comprising an unpleasant tasting, solid core and a flavoured, pharmaceutically acceptable, thin film coating, said coated tablet showing a weight increase of 0.5 to 15 parts per 100 parts by weight of the core, said coating comprising a water-soluble, film-forming polymer, a volatile flavouring agent and a sweetening agent, said coating being capable of masking the unpleasant taste of the core, and said coating having a flavour that is retained in the coating for at least 24 months during storage and that provides a taste perception of said flavour for at least five seconds after oral administration of said tablet, which comprises the steps of :

(a) preparing an aqueous dispersion comprising a film-coating substance, a flavouring agent and a sweetening agent;

(b) placing the uncoated core tablets in a coating pan; and

(c) spray-coating the aqueous dispersion onto the exterior surface of the core tablets at a pan rotation speed and under airflow and temperature conditions sufficient to enable evaporation of water and even-coating of the core tablets.

**2.** A method as claimed in claim 1, wherein the pan is perforated and is rotated at about 8 rpm, the inlet airflow rate is about 1300 cubic feet per minute, the air temperature is about 90 degrees C, and the bed temperature is about 45 degrees C.

**3.** A method as claimed in any of claims 1 to 2, wherein the film-coating substance comprises hydroxypropyl methylcellulose.

**4.** A method as claimed in any of claims 1 to 3, wherein the flavouring agent comprises peppermint flavouring.

**5.** A method as claimed in in any of claims 1 to 4, wherein the flavouring agent comprises fruit flavouring.

**6.** A method as claimed in any of claims 1 to 5, wherein the sweetening agent comprises confectioners sugar.

**7.** A method as claimed in any of claims 1 to 6, wherein the film-forming substance further contains titanium dioxide.

**8.** A method as claimed in any of claims 1 to 7, wherein the tablet core comprises triprolidine hydrochloride.

**9.** A method as claimed in any of claims 1 to 8, wherein the tablet core comprises pseudoephedrine hydrochloride.

**10.** A method as claimed in any of claims 1 to 9 wherein the tablet core comprises triprolidine hydrochloride and pseudoephedrine hydrochloride.

**11.** A method as claimed in any of claims 1 to 10, wherein:

(a) the film-coating substance comprises hydroxypropyl methylcellulose, titanium dioxide and polyethylene glycol; and

(b) the aqueous dispersion is comprised of about 7.5% film-coating substance, about 0.5% flavouring agent, and about 2.5% sweetening agent.

9

EP 0 298 768 B1

**Patentansprüche**
**Patentansprüche für folgende Vertragsstaaten : AT, BE, CH, DE, FR, GB, IT, LI, LU, NL, SE**

1. Pharmazeutische Tablette mit einem unangenehm schmeckenden, festen Kern und einer aromatisierten, pharmazeutisch annehmbaren, dünnen Filmbeschichtung, wobei die beschichtete Tablette eine Gewichtszunahme von 0,5 bis 15 Teilen pro 100 Gew.-Teile des Kerns zeigt, die Beschichtung ein wasserlösliches, filmbildendes Polymer, einen flüchtigen Aromastoff und ein Süßungsmittel umfaßt, die Beschichtung dazu geeignet ist, den unangenehmen Geschmack des Kerns zu maskieren, und die Beschichtung ein Aroma besitzt, das während einer Lagerung von mindestens 24 Monaten in der Beschichtung beibehalten bleibt und eine Geschmackswahrnehmung des Aromas während mindestens 5 Sekunden nach der oralen Verabreichung der Tablette ergibt.

2. Pharmazeutische Tablette nach Anspruch 1, **dadurch gekennzeichnet**, daß die aromatisierte Beschichtung durch Sprühauftrag einer wäßrigen Dispersion, die eine wasserlösliche, pharmazeutisch annehmbare, filmbildende Substanz, einen flüchtigen Aromastoff und ein Süßungsmittel umfaßt, auf die Außenoberfläche der Kerntabletten aufgebracht wird.

3. Pharmazeutische Tablette nach Anspruch 1 oder Anspruch 2, worin die filmbildende Substanz Hydroxypropyl-methylcellulose umfaßt.

4. Pharmazeutische Tablette nach einem der Ansprüche 1 bis 3, worin der Aromastoff Pfefferminzaroma umfaßt.

5. Pharmazeutische Tablette nach einem der Ansprüche 1 bis 3, worin der Aromastoff Fruchtaroma umfaßt.

6. Pharmazeutische Tablette nach einem der Ansprüche 1 bis 5, worin das Süßungsmittel Puderzucker umfaßt.

7. Pharmazeutische Tablette nach einem der Ansprüche 1 bis 6, worin die filmbildende Substanz zusätzlich Titandioxid enthält.

8. Pharmazeutische Tablette nach einem der Ansprüche 1 bis 7, worin der Tablettenkern Triprolidin-hydrochlorid umfaßt.

9. Pharmazeutische Tablette nach einem der Ansprüche 1 bis 7, worin der Tablettenkern Pseudoephedrin-hydrochlorid umfaßt.

10. Pharmazeutische Tablette nach einem der Ansprüche 1 bis 7, worin der Tablettenkern Triprolidin-hydrochlorid und Pseudoephedrin-hydrochlorid umfaßt.

11. Pharmazeutische Tablette nach einem der Ansprüche 2 bis 10, **dadurch gekennzeichnet**, daß
    (a) die filmbildende Substanz Hydroxypropyl-methylcellulose, Titandioxid und Polyethylenglykol umfaßt; und
    (b) die wäßrige Dispersion etwa 7,5% der filmbildenden Substanz, etwa 0,5 % Aromastoff und etwa 2,5 % Süßungsmittel umfaßt.

12. Verfahren zur Herstellung einer aromatisierten, filmbeschichteten pharmazeutischen Tablette nach einem der Ansprüche 2 bis 10, welches darin besteht, pharmazeutische Kerntabletten mit einem dünnen Filmüberzug sprühzubeschichten, **dadurch gekennzeichnet**, daß die Beschichtungsmischung ein wasserlösliches, pharmazeutisch annehmbares, filmbildendes Mittel, einen flüchtigen Aromastoff und ein Süßungsmittel umfaßt.

**Patentansprüche für folgende Vertragsstaaten : ES, GR**

1. Verfahren zur Herstellung einer pharmazeutischen Tablette mit einem unangenehm schmeckenden, festen Kern und einer aromatisierten, pharmazeutisch annehmbaren, dünnen Filmbeschichtung, wobei die beschichtete Tablette eine Gewichtszunahme von 0,5 bis 15 Teilen pro 100 Gew.-Teile des Kerns zeigt, die Beschichtung ein wasserlösliches, filmbildendes Polymer, einen flüchtigen Aromastoff und ein Süßungsmittel umfaßt, die Beschichtung dazu geeignet ist, den unangenehmen Geschmack des Kerns zu maskieren, und die Beschichtung ein Aroma besitzt, das während einer Lagerung von mindestens 24 Monaten in der Beschichtung beibehalten bleibt und eine Geschmackswahrnehmung des Aromas während mindestens 5 Sekunden nach der oralen Verabreichung der Tablette ergibt, welches die folgenden Schritte umfaßt:

(a) Herstellung einer wäßrigen Dispersion, die eine Filmbeschichtungssubstanz, einen Aromastoff und ein Süßungsmittel umfaßt;

(b) die unbeschichteten Kerntabletten in eine Beschichtungspfanne einbringt; und

(c) die wäßrige Dispersion zur Beschichtung auf die äußere Oberfläche der Kerntabletten aufsprüht bei Bedingungen der Pfannendrehgeschwindigkeit, des Luftstroms und der Temperatur, die dazu geeignet sind, ein Verdampfen des Wassers und eine gleichmäßige Beschichtung der Kerntabletten zu ermöglichen.

2.  Verfahren nach Anspruch 1, worin die Pfanne perforiert ist und mit etwa 8 min$^{-1}$ gedreht wird, die Einlaßluftströmung etwa 1300 Kubikfuß pro Minute, die Lufttemperatur etwa 90°C und die Schichttemperatur etwa 45°C betragen.

3.  Verfahren nach einem der Ansprüche 1 bis 2, worin die Filmbeschichtungssubstanz Hydroxypropyl-methylcellulose umfaßt.

4.  Verfahren nach einem der Ansprüche 1 bis 3, worin der Aromastoff Pfefferminzaroma umfaßt.

5.  Verfahren nach einem der Ansprüche 1 bis 4, worin der Aromastoff Fruchtaroma umfaßt.

6.  Verfahren nach einem der Ansprüche 1 bis 5, worin das Süßungsmittel Puderzucker umfaßt.

7.  Verfahren nach einem der Ansprüche 1 bis 6, worin die filmbildende Substanz zusätzlich Titandioxid enthält.

8.  Verfahren nach einem der Ansprüche 1 bis 7, worin der Tablettenkern Triprolidin-hydrochlorid umfaßt.

9.  Verfahren nach einem der Ansprüche 1 bis 8, worin der Tablettenkern Pseudoephedrin-hydrochlorid umfaßt.

10. Verfahren nach einem der Ansprüche 1 bis 9, worin der Tablettenkern Triprolidin-hydrochlorid und Pseudoephedrin-hydrochlorid umfaßt.

11. Verfahren nach einem der Ansprüche 1 bis 10, worin

(a) die filmbildende Substanz Hydroxypropyl-methylcellulose, Titandioxid und Polyethylenglykol umfaßt; und

(b) die wäßrige Dispersion etwa 7,5 % Filmbeschichtungssubstanz, etwa 0,5 % Aromastoff und etwa 2,5 % Süßungsmittel umfaßt.

**Revendications**

**Revendications pour les Etats contractants suivants : AT, BE, CH, DE, FR, GB, IT, LI, LU, NL, SE**

1.  Comprimé pharmaceutique comprenant un corps solide de goût déplaisant et un enrobage pelliculaire fin, pharmaceutiquement acceptable, aromatisé, le comprimé enrobé présentant une augmentation de poids de 0,5 à 15 parties par 100 parties en poids du corps, l'enrobage comprenant un polymère filmogène soluble dans l'eau, un agent aromatisant volatil et agent édulcorant, l'enrobage étant capable de masquer le goût déplaisant du corps, et l'enrobage ayant un arôme qui est retenu dans l'enrobage pendant au moins 24 mois par stockage et qui procure une perception du goût de l'arôme pendant au moins cinq secondes après administration orale du comprimé.

2.  Comprimé pharmaceutique suivant la revendication 1, caractérisé en ce que l'enrobage aromatisé est appliqué par enrobage par pulvérisation d'une dispersion aqueuse comprenant une substance filmogène pharmaceutiquement acceptable, soluble dans l'eau, un agent aromatisant volatil et un agent édulcorant sur la surface extérieure du corps des comprimés.

**3.** Comprimé pharmaceutique suivant la revendication 1 ou 2, où la substance filmogène comprend l'hydroxypropylméthylcellulose.

**4.** Comprimé pharmaceutique suivant l'une quelconque des revendications 1 à 3, où l'agent aromatisant comprend un arôme de menthe.

**5.** Comprimé pharmaceutique suivant l'une quelconque des revendications 1 à 3, où l'agent aromatisant comprend un arôme de fruits.

**6.** Comprimé pharmaceutique suivant l'une quelconque des revendications 1 à 5, où l'agent édulcorant comprend du sucre en poudre.

**7.** Comprimé pharmaceutique suivant l'une quelconque des revendications 1 à 6, où la substance filmogène contient, de plus, du dioxyde de titane.

**8.** Comprimé pharmaceutique suivant l'une quelconque des revendications 1 à 7, où le corps du comprimé comprend du chlorhydrate de triprolidine.

**9.** Comprimé pharmaceutique suivant l'une quelconque des revendications 1 à 7, où le corps du comprimé comprend du chlorhydrate de pseudoéphédrine.

**10.** Comprimé pharmaceutique suivant l'une quelconque des revendications 1 à 7, où le corps du comprimé comprend du chlorhydrate de triprolidine et du chlorhydrate de pseudoéphédrine.

**11.** Comprimé pharmaceutique suivant l'une quelconque des revendications 2 à 10, caractérisé en ce que :
(a) la substance filmogène comprend de l'hydroxypropylméthylcellulose, du dioxyde de titane et du polyéthylèneglycol, et
(b) la dispersion aqueuse comprend environ 7,5% de substance filmogène, environ 0,5% d'agent aromatisant et environ 2,5% d'agent édulcorant.

**12.** Procédé de préparation d'un comprimé pharmaceutique enrobé d'une pellicule aromatisée suivant l'une quelconque des revendications 2 à 10, qui comprend l'enrobage par pulvérisation des corps de comprimés pharmaceutiques avec un enrobage pelliculaire fin, caractérisé en ce que le mélange d'enrobage se compose d'un agent filmogène pharmaceutiquement acceptable, soluble dans l'eau, d'un agent aromatisant volatil et d'un agent édulcorant.

**Revendications pour les Etats contractants suivants : ES, GR**

**1.** Procédé de préparation d'un comprimé pharmaceutique comprenant un corps solide de goût déplaisant et un enrobage pelliculaire fin, pharmaceutiquement acceptable, aromatisé, le comprimé enrobé présentant une augmentation de poids de 0,5 à 15 parties par 100 parties en poids du corps, l'enrobage comprenant un polymère filmogène soluble dans l'eau, un agent aromatisant volatil et un agent édulcorant, l'enrobage étant capable de masquer le goût déplaisant du corps et l'enrobage ayant un arôme qui est retenu dans l'enrobage pendant au moins 24 mois par stockage et qui procure une perception de goût de l'arôme pendant au moins cinq secondes après administration orale des comprimés, qui comprend les étapes de :
(a) préparation d'une dispersion aqueuse comprenant un substance d'enrobage pelliculaire, un agent aromatisant et un agent édulcorant;
(b) le placement des comprimés non enrobés dans une turbine d'enrobage, et
(c) l'enrobage par pulvérisation de la dispersion aqueuse sur la surface extérieure du corps des comprimés à une vitesse de rotation de la turbine et sous un flux d'air et dans des conditions de température suffisantes pour permettre l'évaporation de l'eau et l'enrobage uniforme des corps de comprimés.

**2.** Procédé suivant la revendication 1, où la turbine est perforée et mise en rotation à environ 8 tours par minute, la vitesse d'entrée d'air étant de 36,79 cm par minute (1300 pieds cubes par minute), la température de l'air d'environ 90°C et la température du lit d'environ 45°C.

3. Procédé suivant l'une quelconque des revendications 1 à 2, où la substance d'enrobage pelliculaire comprend de l'hydroxypropylméthylcellulose.

4. Procédé suivant l'une quelconque des revendications 1 à 3, où l'agent aromatisant comprend un arôme de menthe.

5. Procédé suivant l'une quelconque des revendications 1 à 4, où l'agent aromatisant comprend un arôme de fruits.

6. Procédé suivant l'une quelconque des revendications 1 à 5, où l'agent édulcorant comprend du sucre en poudre.

7. Procédé suivant l'une quelconque des revendications 1 à 6, où la substance filmogène contient, de plus, du dioxyde de titane.

8. Procédé suivant l'une quelconque des revendications 1 à 7, où le corps du comprimé comprend du chlorhydrate de triprolidine.

9. Procédé suivant l'une quelconque des revendications 1 à 8, où le corps du comprimé comprend du chlorhydrate de pseudoéphédrine.

10. Procédé suivant l'une quelconque des revendications 1 à 9, où le corps du comprimé comprend du chlorhydrate de triprolidine et du chlorhydrate de pseudoéphédrine.

11. Procédé suivant l'une quelconque des revendications 1 à 10, où
    (a) la substance d'enrobage pelliculaire comprend de l'hydroxypropylméthylcellulose, du dioxyde de titane et du polyéthylèneglycol, et
    (b) la dispersion aqueuse se compose d'environ 7,5% de substance d'enrobage pelliculaire, environ 0,5% d'agent aromatisant et environ 2,5% d'agent édulcorant.